# EUROPEAN PATENT APPLICATION

(11) **EP 4 541 321 A1**
(43) Date of publication of application: **23.04.2025**
(21) Application number: 23204449.5
(22) Date of filing: 18.10.2023
(51) Int. Cl.: A61F 2/38, A61B 17/15, A61B 17/17, A61F 2/46, A61F 2/30

(54) **FEMORAL OFFSET SYSTEM FOR KNEE ARTHROPLASTY PROCEDURE**

(71) Applicant: Aesculap AG, 78532 Tuttlingen (DE)
(72) Inventor: Zouaghi, Housseyn, 52000 CHAUMONT (FR); Moussa, Said, 52000 Chamarandes-Choignes (FR)
(74) Representative: DREISS Patentanwälte PartG mbB

(57) **Abstract**

The disclosure refers to a femoral offset system (14) for use in a knee arthroplasty procedure, in particular a revision procedure, comprising a femur component (12) being connectable to a distal portion of a femur, and an femoral offset instrument (10) being connectable to the femur component (12), wherein the femoral offset instrument (10) comprises a femur support (16), the femur support (16) being adapted to be connected to the femur component (12) via a connection mechanism.

## Description

The disclosure refers to a femoral offset system for use in a knee arthroplasty procedure, in particular a revision procedure comprising a femoral offset instrument and to such a femoral offset instrument. A femoral offset instrument allows to determine and/or measure and or check a femoral offset of a femoral implant on a distal portion of a femur bone. Such instruments are known from prior art.

The purpose of the present disclosure is to provide a femoral offset system, which allows comfortable handling thereby simplifying the workflow when using the femoral offset system in a knee arthroplasty procedure.

### Disclosure of the invention

A first aspect refers to a femoral offset system for use in a knee arthroplasty procedure, in particular a revision procedure. The femoral offset system comprises a femur component being connectable to a distal portion of a femur, and a femoral offset instrument being connectable to the femur component. The femoral offset instrument comprises a femur support, the femur support being adapted to be connected to the femur component via a connection mechanism. The connection mechanism for connecting the femoral offset instrument to the femur component comprises: a fixed peg and a movable peg connected to the femoral offset instrument, a manually operable operating element, which allows retracting the movable peg by applying a manual force against a tension force, and receiving pockets for the fixed peg and the movable peg arranged on the femur component. For connecting the femoral offset instrument to the femur component, the movable peg is retracted by applying a manual force against a tension force, the femoral offset instrument and the femur component are aligned to each other accordingly, the manual force is released and the movable peg snaps into the respective receiving pocket due to the acting tension force.

The connection mechanism allows to connect the femoral offset instrument to the femur component in a fixedly, statically, non-rotationally and undisplaceable but releasable manner.

The proposed solution requires only manually pressing operating element and thereby retracting the movable peg by applying a manual force against a tension force and releasing the operating element. In particular, the proposed solution avoids the use of screws or other connection variants, which require more effort.

According to an embodiment, the fixed peg and the movable peg of the femoral offset instrument are arranged on the femur support medially and laterally with regard to the marking element. The fixed peg is fixedly connected to the femur support. The movable peg is arranged on the femur support in a movable manner.

Advantageously, the movable peg and the operating element are fixedly connected. The movable peg and the operating element might be fixedly connected via firmly bonding or other suitable connection manners. The movable peg and the manually operable operating element might be realized as an integrally component.

In some exemplary embodiments, the femur support comprises at least one guiding groove, and the operating element comprises a corresponding protrusion arranged in said guiding groove, wherein the femur support and the operating element are adapted such that the protrusion can slide in the guiding groove. When operating the operating element by applying a manual force or releasing the manual force, the operating element moves in the respective direction in a translatory manner guided by the guiding groove.

The two guiding grooves and the two protrusions may each be arranged distally and proximally relative to each other.

Advantageously, the operating element is secured to the femur support, for example via a suitable securing mechanism. Despite the securing, the operating element and the femur support are movable relative to each other to a given extent. The securing might be done via a pin or two pins located in corresponding recesses of the femur support, the two pins preventing the protrusion(s) from falling out of the guiding groove(s).

According to further preferred embodiments, the femur support comprises at least one femur attachment member for fixedly connecting the femur support to the femur component and the femur component comprises at least one corresponding recess for receiving the one femur attachment member. For example, the femur attachment member extends medially or laterally from the femur support.

Advantageously, the femur support comprises two femur attachment members, the two femur attachment members being medially and laterally arranged with regard to the marking element, wherein the fixed peg is fixedly connected to one of the attachment member and the other one of the attachment members receives the operating element. For example, the medially arranged femur attachment member extends medially from the femur support. For example, the laterally arranged femur attachment member extends laterally from the femur support. In case the fixed peg is fixedly connected to the medially arranged femur attachment member, the fixed peg extends medially therefrom. In case the fixed peg is fixedly connected to the laterally arranged femur attachment member, the fixed peg extends laterally therefrom.

In case the laterally arranged attachment member receives the operating element, the tension force acts in the lateral direction, and the movable peg connected to the operating element extends laterally from the laterally arranged attachment member in case no manual force is applied against the tension force.

In case the medially arranged attachment member receives the operating element, the tension force acts in the medial direction, and the movable peg connected to the operating element extends medially from the medially arranged attachment member in case no manual force is applied against the tension force.

The femur component might comprise two corresponding recesses for receiving the two femur attachment members, the recesses being medially and laterally arranged with regard to each other, wherein the receiving pockets for receiving the fixed pin and the movable pin extend from the recess in the medial and lateral directions, respectively.

According to an example, the femur component is a trial femur implant and/or a femoral cutting guide. The femur component, in particular the trial femur implant or the femoral cutting guide is, temporarily connectable during knee arthroplasty procedure.

According to an example, the marking element is an offset eyeballing. The marking element allows eyeballing of the femoral offset. Combined with an offset bushing, the marking element allows to determine and/or measure and/or check a femoral offset. The offset bushing is a separate component, which is connectable to the femoral offset instrument. Held by the femur support, the offset bushing is rotatable around a rotational axis. According to a further example, the marking element and the offset bushing may allow guiding of a drill for preparing the cone of the femur using a drill.

A second aspect of the disclosure refers to a femoral offset instrument for a femoral offset system according to the described embodiments, the femoral offset instrument comprising a femur support, the femur support being adapted to be connected to the femur component via a connection mechanism, wherein the connection mechanism comprises:
a fixed peg and a movable peg connected to the femoral offset instrument, a manually operable operating element, which allows retracting the movable peg by applying a manual force against a tension force.

According to further embodiments, the femoral offset instrument might be designed as described above with regard to the femoral offset system.

A third aspect of the disclosure refers to a femur component for a femoral offset system according to the described embodiments, the femur component being connectable to a distal portion of a femur and comprising receiving pockets for a fixed peg and a movable peg of the femoral offset instrument.

According to further embodiments, the femur component might be designed as described above with regard to the femoral offset system.

A fourth aspect of the disclosure refers a set of femoral offset systems and/or femoral offset instruments and/or femur components, comprising at least two femoral offset systems according to the described embodiments and/or at least two femur components according to the described embodiments and/or at least two femur components according to the described embodiments, wherein the at least two femoral offset systems and/or the at least two femur components and/or the two femur components differ in at least a size or a form.

Further advantageous embodiments are derivable from the following description of Exemplary embodiments with reference to the drawings. In the drawings:
- Fig. 1: schematically depicts a femoral offset instrument in different views (a) and b) and c));

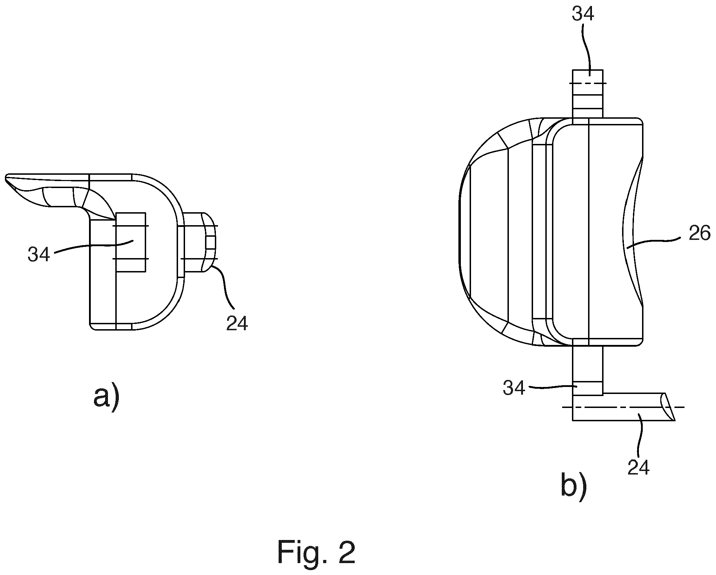

- Fig. 2: schematically depicts a part of a femoral offset instrument in different views (a) and b));
- Fig. 3: schematically depicts a femur component, according to an embodiment in different views (a) and b));
- Fig. 4: schematically depicts a femoral offset system according to an embodiment in different views (a) and b));
- Fig. 5: schematically depicts a femur component, according to another embodiment in different views (a) and b));
- Fig. 6: schematically depicts a femoral offset system according to another.

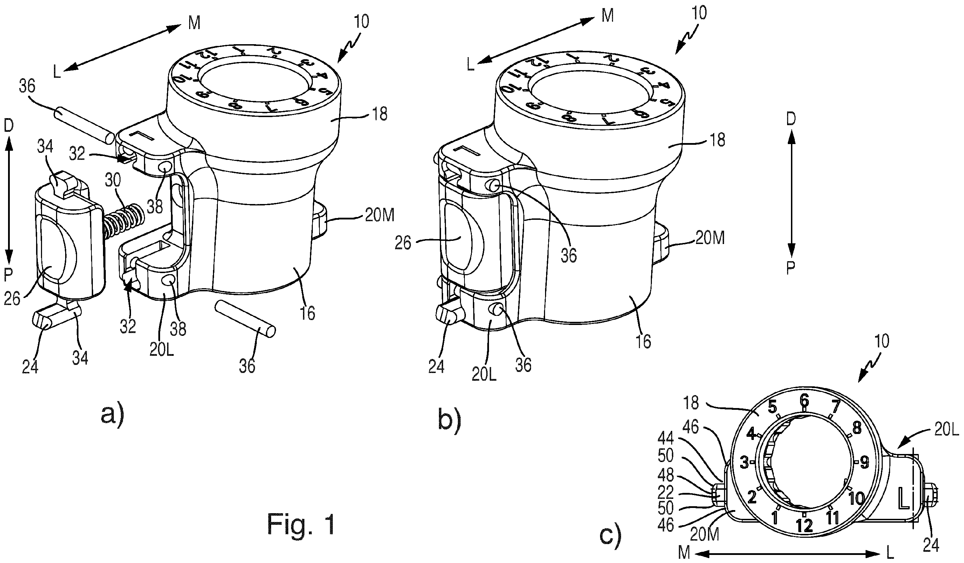

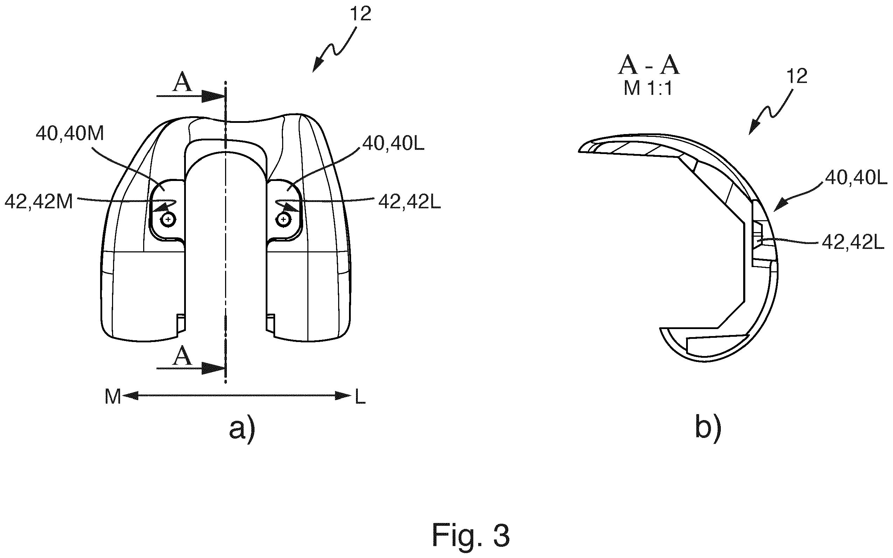

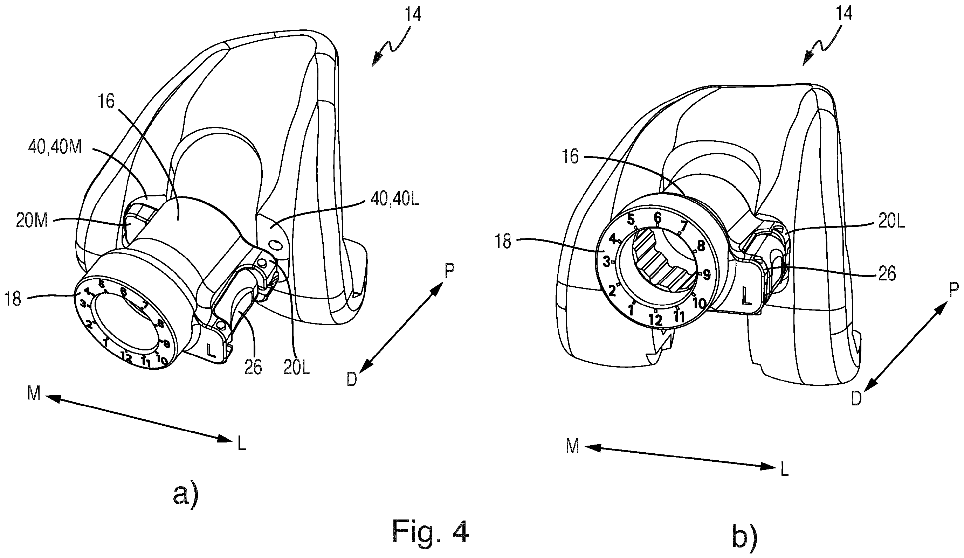

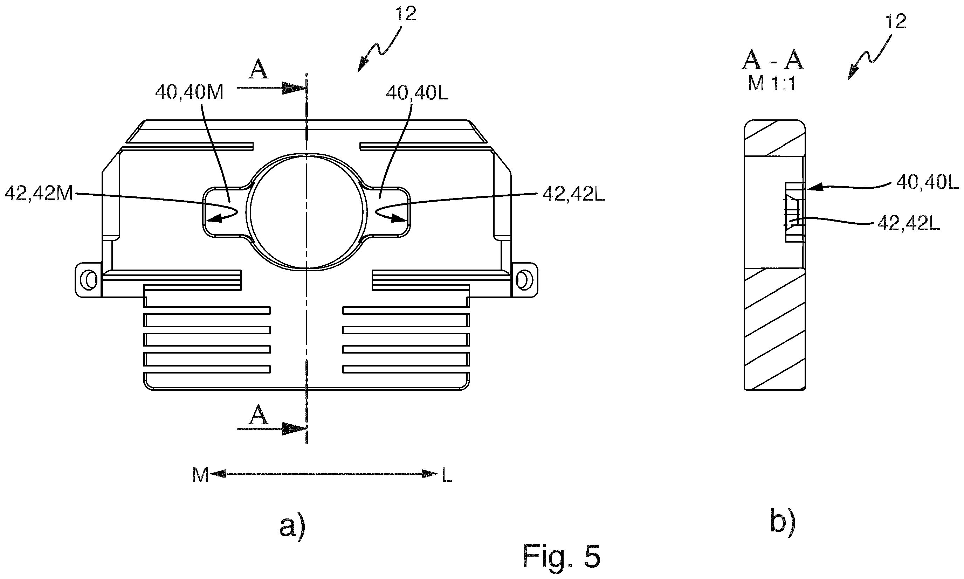

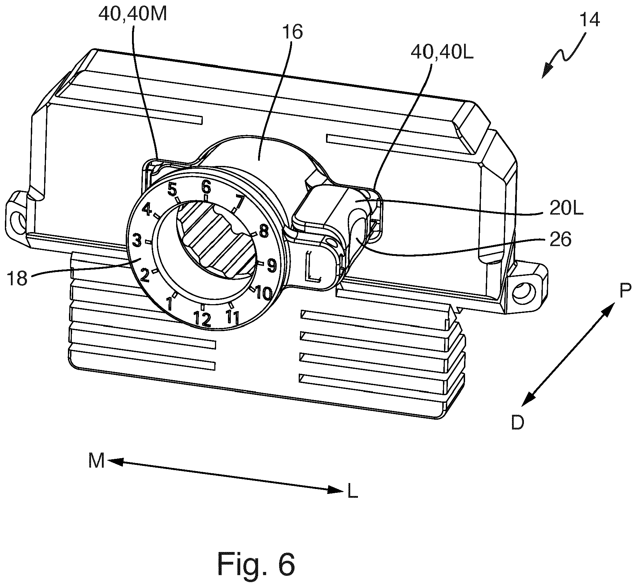

Figure 1 schematically depicts a femoral offset instrument 10. The femoral offset instrument 10 might be used together with a femur component 12 as depicted for example in figures 3 and 5 in femoral offset system 14 as depicted for example in figures 4 and 6.

The femoral offset system 14 is designed for use in a knee arthroplasty procedure, in particular a revision procedure.

The femoral offset instrument 10 is right or left specific instrument with regard to a right or left femur. This means the femoral offset instrument 10 comprises a right or left orientation, which is indicated for example by an "R" or "L" displayed on the instrument. The following examples refer to a left specific instrument.

A distal, proximal, medial, lateral direction with regard to a left femur is indicated in the figures by respective arrows D/P, M/L.

The femur component 12 is temporarily connectable to a distal portion of a femur (not displayed).

The femoral offset instrument 10 is connectable to the femur component 12, see for example figures 4 and 6 via a connection mechanism. The connection mechanism allows to connect the femoral offset instrument 10 to the femur component 12 in a fixedly, statically, non-rotationally and undisplaceable but releasable manner. The design and functionality is described in the following in an exemplary manner with regard to the figures.

The femoral offset instrument 10 comprises a femur support 16. The femur support 16 is adapted to be connected to the femur component 12 via the connection mechanism. According to the examples, the femoral offset instrument 10 comprises a marking element 18, which provides marking of the offset.

Fig. 1 a) depicts the femoral offset instrument 10 in an unmounted state and in an exploded view. Fig. 1 b) and c) depict the femoral offset instrument 10 in a mounted state.

In the example, the femur support 16 comprises two femur attachment members 20 for fixedly connecting the femur support 16 to the femur component 12. A medially arranged femur attachment member 20M extends medially from the femur support 16. A laterally arranged femur attachment member 20L extends laterally from the femur support 16.

Each attachment member 20 may have a flat end 44 combined with two rounded ends 46, making it easier to position the attachment member 20 and offering greater precision when positioning.

The femoral offset instrument 10 comprises a fixed peg 22 and a movable peg 24, both connected to the femoral offset instrument 10. The fixed peg 22 is fixedly connected to the femur support 16. In the example, the fixed peg 22 is connected to the medially arranged femur attachment member 20M. The movable peg 24 is arranged on the femur support 16 in a movable manner.

The fixed peg 22 may have a flat 48 end combined with two rounded ends 50, making it easier to position the fixed peg 22 and offering greater precision when positioning.

In the example, the movable peg 24 is fixedly connected to an operating element 26 of the femoral offset instrument 10. According to the example, the movable peg 24 and the operating element 26 are realized as an integrally component. In the example, the laterally arranged femur attachment member 20L receives the operating element 26.

Therefore, the fixed peg 22 and the movable peg 24 of the femoral offset instrument 10 are arranged on the femur support 16 medially and laterally with regard to the marking element 18.

The operating element 26 is manually operable, which allows retracting the movable peg 24 by applying a manual force against a tension force. In the example, the tension force is provided by spring 30. According to the example, the tension force acts in the lateral direction. The movable peg connected to the operating element 26 extends laterally from the laterally arranged attachment member 20L in case no manual force is applied against the tension force, see fig. 1. The movable peg can be retracted in the medial directing by applying a manual force on the operating element 26 against a tension force.

In the example, the femur support 16 comprises two guiding grooves 32. The operating element 26 comprises two corresponding protrusions 34, wherein one protrusion is formed by the movable peg 24. The protrusions 34 are arranged in a respective guiding groove 32 each, wherein the femur support 16 and the operating element 26 are adapted such that the protrusions 34 might slide in the guiding grooves 32. When operating the operating element 26 by applying a manual force or releasing the manual force, the operating element moves in the respective direction in a translatory manner guided by the guiding grooves 32. In the example, the two guiding grooves 32 and the two protrusions 34 are arranged distally and proximally relative to each other.

In the example, the operating element 26 is secured to the femur support 16, for example via a suitable securing mechanism. Despite the securing, the operating element 26 and the femur support 16 are movable relative to each other to a given extent. The securing is done via two pins 36 located in corresponding recesses 38 of the femur support 16, the two pins 36 preventing the protrusions 34 from dropping out of the guiding grooves 32.

With regard to the femur component 12, the connection mechanism comprises two recesses 40 arranged on the femur component 12 for receiving corresponding femur attachment members 20. The recesses 40M, 40L are medially and laterally arranged with regard to each other.

The femur component 12 comprises further two receiving pockets 42, one for the fixed peg 22 and one for the movable peg 24. The receiving pocket 42M for receiving the fixed pin 22 extends from the medially arranged recess 40M in the medial direction and the receiving pocket 42L for receiving the movable pin 24 extends from the laterally arranged recess 40 L in the lateral direction.

For connecting the femoral offset instrument to the femur component, the following steps might be performed:
The femoral offset instrument and the femur component are positioned accordingly to each other, by positioning the medially arranged attachment member 20M in the medially arranged recess 40M such that the fixed peg 22 engages with the receiving pocket 42M.

The movable peg 24 is retracted by applying a manual force on the operating element 26 against the tension force, such that the movable peg 24 is retracted in the medial direction.

The femoral offset instrument 10 and the femur component 12 are aligned to each other accordingly by positioning the laterally arranged attachment member 20L in the recess 40L, and the manual force is released and the movable peg 24 snaps in the lateral direction into the respective receiving pocket 42 L due to the acting tension force.

The proposed solution requires only manually pressing the operating element 26 and thereby retracting the movable peg 24 by applying a manual force against the tension force and releasing the operating element 26. In particular, the proposed solution avoids the use of screws or other connection variants, which require more effort.

According to an example, the femur component is a trial femur implant, see fig. 3 and 4.

According to a further example, the femur component is a femoral cutting guide, see fig. 5 and 6.

According to the examples, the marking element 18 is an offset eyeballing, which allows eyeballing of the femoral offset. During knee arthroplasty procedure, the offset eyeballing helps the surgeon to determine the femoral offset to be used for positioning of an implant. Combined with an offset bushing, which is not displayed in the figures, the marking element 18 allows determine and/or measure and/or check the correct femoral offset. The offset bushing is a separate component, which is connectable to the femoral offset instrument. Held by the femur support 16, the offset bushing is rotatable around a rotational axis. According to a further example, the marking element 18 and the offset bushing may allow guiding of a drill for preparing the cone of the femur using a drill.

## Claims

1. Femoral offset system (14) for use in a knee arthroplasty procedure, in particular a revision procedure, comprising a femur component (12) being connectable to a distal portion of a femur, and an femoral offset instrument (10) being connectable to the femur component (12), wherein the femoral offset instrument (10) comprises a femur support (16), the femur support (16) being adapted to be connected to the femur component (12) via a connection mechanism, wherein the connection mechanism comprises:
a fixed peg (22) and a movable peg (24) connected to the femoral offset instrument (10), a manually operable operating element (26), which allows retracting the movable peg (24) by applying a manual force against a tension force, and receiving pockets (42, 42M, 42L) for the fixed peg (22) and the movable peg (24) arranged on the femur component (12).

2. Femoral offset system (14) according to claim 1,
wherein the fixed peg (22) and the movable peg (24) of the femoral offset instrument (10) are arranged on the femur support (16) medially and laterally with regard to a marking element (18) of the femoral offset instrument (10).

3. Femoral offset system (14) according to any of the claims 1 or 2, wherein the movable peg (24) and the operating element (26) are fixedly connected, in particular the movable peg (24) and the operating element (26) are realized as an integrally component.

4. Femoral offset system (14) according to any of the preceding claims, wherein the femur support (16) comprises at least one guiding groove (32), and the operating element (26) comprises a corresponding protrusion (34) arranged in said guiding groove (32), wherein the femur support (16) and the operating element (26) are adapted such that the protrusion (34) is adapted to slide in the guiding groove (32).

5. Femoral offset system (14) according to claim 4,
wherein the movable peg (24) forms a protrusion (34) arranged in said guiding groove (32).

6. Femoral offset system (14) according to any of the preceding claims, wherein the operating element (26) is secured to the femur support (16).

7. Femoral offset system (14) according to any of the preceding claims, wherein the femur support (16) comprises at least one femur attachment member (20) for fixedly connecting the femur support (16) to the femur component (12) and the femur component (12) comprises at least one corresponding recess (40) for receiving the one femur attachment member (20).

8. Femoral offset system (14) according to claim 7,
wherein the femur support comprises two femur attachment members (20, 20M, 20L), the two femur attachment members being medially and laterally arranged with regard to the marking element (18), wherein the fixed peg (22) is fixedly connected to one of the attachment members (20M) and the other one of the attachment members (20L) receives the operating element (26).

9. Femoral offset system (14) according to claim 8,
wherein the femur component (12) comprises two corresponding recesses (40, 40M, 40L) for receiving the two femur attachment members (20, 20M, 20L), the recesses (40, 40M, 40L) being medially and laterally arranged with regard to each other, wherein the receiving pockets (42, 42M, 42L) for receiving the fixed pin (22) and the movable pin (24) extend from the recesses (40, 40M, 40L) in the medial and lateral directions, respectively.

10. Femoral offset system (14) according to any of the preceding claims, wherein the femur component (12) is a trial femur implant and/or a femoral cutting guide.

11. Femoral offset system (14) according to any of the preceding claims, wherein the marking element (18) is an offset eyeballing.

12. Femoral offset instrument (10) for a femoral offset system (14) according to any of the preceding claims, the femoral offset instrument (10) comprising a femur support (16), the femur support (16) being adapted to be connected to the femur component (12) via a connection mechanism, wherein the connection mechanism comprises:
a fixed peg (22) and a movable peg (24) connected to the femoral offset instrument (10), a manually operable operating element (26), which allows retracting the movable peg (22) by applying a manual force against a tension force.

13. Femur component (12) for a femoral offset system (14) according to any of the preceding claims 1 to 11, the femur component (12) being connectable to a distal portion of a femur and comprising receiving pockets (42, 42M, 42L) for a fixed peg (22) and a movable peg (24) of a femoral offset instrument (10).

14. A set of femoral offset systems (14) and/or femoral offset instruments (10) and/or femur components (12), comprising at least two femoral offset systems (14) according to any of the claims 1 to 11 and/or at least two femur components (12) according to claim 12 and/or at least two femur components (12) according to claim 13, wherein the at least two femoral offset systems (14) and/or the at least two femur components (12) and/or the two femur components (12) differ in at least a size or a form.
